# EUROPÄISCHE PATENTANMELDUNG

(11) **EP 2 525 228 A1**
(43) Veröffentlichungstag der Anmeldung: **21.11.2012**
(21) Anmeldenummer: 11166477.7
(22) Anmeldetag: 18.05.2011
(51) Int. Cl.: G01N 33/86

(54) **Verfahren zur Bestimmung des Risikos einer Clopidogrel-Resistenz**

(71) Anmelder: Siemens Healthcare Diagnostics Products GmbH, 35041 Marburg (DE)
(72) Erfinder: Rechner, Andreas, Dr., 35041 Marburg (DE)

(57) **Zusammenfassung**

Die vorliegende Erfindung betrifft ein Verfahren zur Identifizierung von Patienten, die mit hoher Wahrscheinlichkeit nicht von einer Therapie mit Clopidogrel profitieren werden. Es wurde gefunden, dass anhand der Thrombozytenaktivität eines Patienten bereits vor der Einnahme von Clopidogrel festgestellt werden kann, ob für den Patienten ein erhöhtes Risiko einer Clopidogrel-Resistenz ("high on-treatment platelet reactivity") besteht.

## Beschreibung

Die vorliegende Erfindung liegt auf dem Gebiet der Diagnostik von kardiovaskulären und thrombotischen Erkrankungen, die eine individualisierte Therapie zum Ziel hat. Die Erfindung betrifft insbesondere ein Verfahren zur Identifizierung von Patienten, die mit hoher Wahrscheinlichkeit nicht von einer Therapie mit Clopidogrel profitieren werden.

Clopidogrel (Handelsnamen: Plavix^{®}, Iscover^{®}) ist ein Arzneistoff, der die Blutgerinnung hemmt und der zunehmend zur Routinebehandlung von Patienten eingesetzt wird, die an einer akuten kardiovaskulären oder thrombotischen Erkrankungen, wie Herzinfarkt oder Schlaganfall leiden, oder die ein solches Ereignis erlitten haben und weiterhin prophylaktisch behandelt werden sollen, um das Risiko eines wiederkehrenden thrombotischen Ereignisses zu verringern. Auch beispielsweise im Rahmen der Behandlung von Patienten, die eine Stent-Implantation in ein Herzkranzgefäß erhalten, wird Clopidogrel verabreicht, um die sogenannten Stentthrombosen zu vermeiden.

Clopidogrel wirkt als sog. Plättchenhemmer oder Thrombozyteninhibitor, weil es den ADP-Rezeptor auf den Thrombozyten irreversibel hemmt und damit die Aktivierung und die Aggregation der Thrombozyten inhibiert. Häufig wird Clopidogrel kombiniert mit Acetylsalicylsäure (ASS), einem anderen Thrombozyteninhibitor, verabreicht, da durch diese Behandlung eine signifikante Verringerung eines Schlaganfalloder Herzinfarktrisikos erzielt wird.

Es ist allerdings bekannt, dass bei zahlreichen mit Clopidogrel-behandelten Patienten nicht die erwünschte Wirkung der Hemmung der Thrombozytenaktivität erreicht wird. Dieses Nicht-Ansprechen auf eine Clopidogrel-Therapie wird auch als Clopidogrel-Resistenz oder auch als "high on-treatment platelet reactivity" (HPR) bezeichnet (Bonello, L. et al., Consensus and future directions on the definition of high on-treatment platelet reactivity to adenosine diphosphate. J Am Coll Cardiol. 2010 Sep 14;56(12): 919-33). Die Ursachen dieses Wirkungsversagens sind unbekannt.

Die Behandlung Clopidogrel-resistenter Patienten mit Clopidogrel hat zur Folge, dass für diese Patienten trotz Therapie ein unvermindert hohes Risiko für ein erneutes thrombotisches Ereignis besteht, solange bis die Clopidogrel-Resistenz z.B. mittels eines Thrombozytenfunktionstests diagnostiziert wird, und die Therapie auf ein anderes Präparat umgestellt wird. Um Patienten mit Clopidogrel-Resistenz optimal vor einem erneuten thrombotischen Ereignis zu schützen, müssten diese Patienten von Anfang an mit anderen Thrombozytenhemmern behandelt werden. Allerdings ist bislang kein Verfahren bekannt, mit dem eine Clopidogrel-Resistenz für einen Patienten diagnostiziert werden könnte, ohne dass der Patient tatsächlich mit Clopidogrel behandelt und ein Wirkungsversagen festgestellt wurde.

Es ist daher wünschenswert, über ein Verfahren zu verfügen, das es ermöglicht, solche Patienten zu identifizieren, die höchstwahrscheinlich Clopidogrel-resistent sind. Dies hätte den Vorteil, dass überhaupt keine Behandlung mit Clopidogrel durchgeführt werden müsste, sondern von Anfang an ein alternativer Thrombozytenhemmer verabreicht werden könnte. Dies wiederum hätte den Vorteil, dass ein Clopidogrel-resistenter Patient von Anfang an gezielt mit anderen Thrombozyteninhibitoren wirksam therapiert werden kann, wodurch sich sein Risiko für ein erneutes thrombotisches Ereignis von Anfang an verringern würde.

Der vorliegenden Erfindung liegt daher die Aufgabe zugrunde, ein Verfahren bereit zu stellen, das es ermöglicht, für einen Patienten, für den eine Therapie mit Thrombozytenhemmern vorgesehen ist, das Risiko des Nicht-Ansprechens auf eine Behandlung mit Clopidogrel zu beurteilen.

Diese Aufgabe wird dadurch gelöst, dass zu einem Zeitpunkt, in dem der Patient noch kein Clopidogrel eingenommen hat, die Thrombozytenaktivität in einer Probe des Patienten bestimmt wird. Es wurde gefunden, dass Patienten, die eine erhöhte Thrombozytenaktivität aufweisen, ein erhöhtes Risiko einer Clopidogrel-Resistenz haben.

Gegenstand der vorliegenden Erfindung ist daher ein Verfahren zur Bestimmung des Risikos einer Clopidogrel-Resistenz eines Patienten, wobei das Verfahren folgende Schritte umfasst:
a) Messung der Thrombozytenaktivität in einer Probe des Patienten zu einem Zeitpunkt, in dem der Patient noch kein Clopidogrel eingenommen hat; und
b) Vergleich der gemessenen Thrombozytenaktivität mit einer statistisch ermittelten Entscheidungsgrenze,
wobei eine erhöhte Thrombozytenfunktion, die jenseits der Entscheidungsgrenze liegt, ein erhöhtes Risiko einer Clopidogrel-Resistenz des Patienten anzeigt.

Im Sinne der vorliegenden Erfindung umfasst der Begriff "Clopidogrel" den Wirkstoff Methyl-(*S*)-α-(2-chlorphenyl)-6,7-dihydrothieno[3,2-c]pyridin-5(4*H*)-acetat sowie dessen pharmazeutisch wirksamen Salze, wie z.B. das Hydrogensulfat, das Besilat (Benzolsulfonat) oder das Hydrochlorid.

Unter dem Begriff "Clopidogrel-Resistenz" ist ein Nicht-Ansprechen eines Patienten auf eine Clopidogrel-Therapie bzw. ein Wirkungsversagen einer Clopidogrel-Therapie bei einem Patienten zu verstehen. Eine Clopidogrel-Resistenz liegt dann vor, wenn nach der Verabreichung von Clopidogrel nicht die erwünschte Wirkung erreicht wird, nämlich eine messbare Hemmung der Thrombozytenaktivität.

Indikationen für eine Therapie mit Clopidogrel sind Erkrankungen, die mit einer erhöhten Thrombosegefahr einhergehen, wie kardiovaskuläre oder thrombotische Erkrankungen wie z.B. Herzinfarkt, instabile Angina pectoris, ischämischer Schlaganfall und periphere arterielle Verschlusserkrankungen, sowie die Implantation von Gefäßstützen (Stents) in Koronararterien.

Das erfindungsgemäße Verfahren eignet sich daher bevorzugt zur Bestimmung des Risikos einer Clopidogrel-Resistenz bei Patienten, die an einer der vorgenannten Erkrankungen leiden und/oder für die eine Stent-Implantation vorgesehen ist und für die deshalb eine antithrombotische Therapie mit Clopidogrel zur Disposition steht.

Die Messung der Thrombozytenaktivität erfolgt mit Hilfe eines Thrombozytenfunktionstests. Zu Bestimmung der Thrombozytenfunktion sind eine Reihe unterschiedlicher Testformate bekannt, z.B. verschiedene Thrombozytenaggregationsteste. Je nach Testformat kann die Thrombozytenfunktion in einer Vollblutprobe oder in einer Probe plättchenreichen Plasmas (PRP-Probe) des Patienten bestimmt werden.

In einer Ausführungsform des erfindungsgemäßen Verfahrens umfasst die Messung der Thrombozytenaktivität das Inkontaktbringen der Patientenprobe, bevorzugt eine Vollblutoder PRP-Probe, mit mindestens einem Thrombozytenaktivator, bevorzugt mit mindestens einem Thrombozytenaktivator aus der Gruppe ADP (Adenosin-5'-diphosphat), Kollagen, Epinephrin, Arachidonsäure, Ristocetin und Thrombin. Unter dem Begriff Thrombozytenaktivator ist eine Substanz zu verstehen, die die Aggregation von Thrombozyten induzieren kann. Bevorzugterweise wird die Probe mit einer Kombination von Thrombozytenaktivatoren in Kontakt gebracht, bevorzugt mit einer Kombination von Kollagen und ADP (Kol/ADP) oder mit einer Kombination von Kollagen und Epinephrin (Kol/Epi).

In einer weiteren Ausführungsform des erfindungsgemäßen Verfahrens umfasst die Messung der Thrombozytenaktivität neben dem Inkontaktbringen der Patientenprobe mit mindestens einem Thrombozytenaktivator zusätzlich das Inkontaktbringen der Patientenprobe mit einem Aktivator von intrazellulären Adenylatzyklasen, bevorzugterweise mit einem Aktivator von intrazellulären Adenylatzyklasen aus der Gruppe Prostaglandin E1 (PGE 1), Forskolin, Prostaglandin I2, Iloprost und Cicaprost.

Die Messung der Thrombozytenaktivität kann dann beispielsweise durch die Bestimmung der Geschwindigkeit der Thrombozytenaggregatbildung in plättchenreichem Plasma oder durch die Bestimmung der maximalen Aggregationsmasse der Thrombozyten, z.B. mittels Lichttransmissionsaggregometrie bestimmt werden (Plättchenaggregation nach Born).

In einer bevorzugten Ausführungsform des erfindungsgemäßen Verfahrens findet die Messung der Thrombozytenaktivität in Vollblut und unter Flussbedingungen und damit in Gegenwart von hohen Scherkräften statt. Ein solches Testprinzip ist beispielsweise im Platelet Function Analyzer System (PFA-100^{®}, PFA-200, Siemens Healthcare Diagnostics GmbH, Marburg, Deutschland) verwirklicht.

Zur Simulation der Flussbedingungen und der Scherkräfte, wie sie in kleineren arteriellen Blutgefäßen herrschen, wird in einer speziellen Messzelle ein Unterdruck von etwa -40mbar erzeugt und das Citratvollblut, dass sich in einem Probenreservoir befindet, strömt durch eine Kapillare, die einen Durchmesser von etwa 100-200 µm hat. Die Kapillare mündet in eine Messkammer, die mit einem Trennelement, z.B. mit einer Membran, abgeschlossen ist, welche eine kapillare zentrale Öffnung (Apertur) enthält, durch die das Blut aufgrund des Unterdrucks durchtritt. In den meisten Fällen ist die Membran zumindest in dem Bereich um die Apertur mit einem oder mehreren Aktivatoren, die die Thrombozytenaggregation induzieren, versetzt, so dass das vorbeiströmende Blut mit den aggregationsinduzierenden Substanzen im Bereich der Apertur in Kontakt kommt. Infolge der induzierten Adhäsion und Aggregation der Thrombozyten bildet sich im Bereich der Apertur ein Thrombozytenpfropf (Thrombus), der die Membranöffnung verschließt und den Blutfluss stoppt. In diesem System wird üblicherweise die Zeit gemessen, die bis zum Verschluss der Membranöffnung benötigt wird. Diese sogenannte Verschlusszeit (CT) korreliert mit der Funktionsfähigkeit der Thrombozyten. Eine Messzelle zur Verwendung in einem Verfahren zu Bestimmung der Thrombozytenfunktion anhand der Verschlusszeit ist z.B. in dem Patentdokument WO 97/34698 beschrieben. Bevorzugte Messzellen sind mit einer Membran ausgestattet sind, die mit Kollagen (Kol) und zusätzlich entweder mit ADP oder mit Epinephrin (Epi) beschichtet ist oder die mit ADP und Prostaglandin E1 (PGE1) beschichtet ist. Verschiedene Trennelemente sowie deren Herstellung und Verwendung sind z.B. in dem Patentdokument EP-B1-716744 oder in EP-A1-1850134 beschrieben.

In einer bevorzugten Ausführungsform des erfindungsgemäßen Verfahrens umfasst die Messung der Thrombozytenaktivität also das Durchleiten der Probe, bevorzugt einer Vollblutprobe, durch eine Kapillare und anschließend durch eine Öffnung eines Trennelements und Messung der Zeit, die für die Bildung eines Thrombozytenpfropfs an der Öffnung des Trennelements bis zum Verschluss der Öffnung benötigt wird.

Bevorzugterweise enthält das Trennelement mindestens einen Thrombozytenaktivator, z.B. aus der Gruppe ADP (Adenosin-5`-diphosphat), Kollagen, Epinephrin, Arachidonsäure, Ristocetin und Thrombin. Besonders bevorzugt enthält das Trennelement eine Kombination von Thrombozytenaktivatoren, insbesondere eine Kombination von Kollagen und ADP (Kol/ADP) oder eine Kombination von Kollagen und Epinephrin (Kol/Epi). Optional kann das Trennelement zusätzlich einen Aktivator von intrazellulären Adenylatzyklasen enthalten, bevorzugterweise einen Aktivator von intrazellulären Adenylatzyklasen aus der Gruppe Prostaglandin E1 (PGE 1), Forskolin, Prostaglandin I2, Iloprost und Cicaprost.

In einer bevorzugten Ausführungsform des erfindungsgemäßen Verfahrens umfasst die Messung der Thrombozytenaktivität die Verwendung einer Vorrichtung, wobei die Vorrichtung folgende Elemente enthält:
- eine Haltekammer zum Halten der Probe;
- eine Kapillare, durch die die Probe von der Haltekammer in eine Messkammer geleitet wird;
- eine Messkammer, die durch ein Trennelement in zwei Kompartimente unterteilt ist, wobei das erste Kompartiment die Probe aus der Kapillare aufnimmt;
- ein Trennelement, das die Messkammer in zwei Kompartimente unterteilt und das eine Öffnung aufweist, durch welche die Probe aus dem ersten Kompartiment in das zweite Kompartiment fließen kann.

Das Trennelement einer solchen Vorrichtung enthält mindestens einen Thrombozytenaktivator, bevorzugt eine der oben beschriebenen Kombinationen von Thrombozytenaktivatoren. Es kann zusätzlich einen der oben genannten Aktivatoren von intrazellulären Adenylatzyklasen enthalten.

Unabhängig davon mit welchem Testprinzip die
Thrombozytenaktivität gemessen wird, wird gemäß der vorliegenden Erfindung die in einer Probe des Patienten gemessene Thrombozytenaktivität mit einer statistisch ermittelten Entscheidungsgrenze (auch Cut-off Wert) verglichen.

Für die Ermittlung der Entscheidungsgrenze kann beispielsweise das statistische Verfahren der Analyse der Receiver Operating Characteristic Kurve (ROC Kurve) angewendet werden. Mit dieser Methode lässt sich basierend auf Daten, bei denen beim selben Patienten sowohl vor wie auch nach der Einnahme von Clopidogrel die Thrombozytenfunktion bestimmt wurde, eine optimale Entscheidungsgrenze für eine Zwei-Klassen-Klassifizierung berechnen, die eine Unterscheidung von Patienten mit einem erhöhten Risiko einer Clopidogrel-Resistenz von Patienten mit einem deutlich geringeren Risiko einer Clopidogrel-Resistenz ermöglicht. Hierzu sollten die Ergebnisse von mindestens 60, besser 120 oder mehr Patienten in die Berechnung einbezogen werden.

Unter einem "erhöhten Risiko einer Clopidogrel-Resistenz" ist zu verstehen, dass die Wahrscheinlichkeit, dass Clopidogrel bei einem Patienten nicht wirkt, etwa 2 - 4-fach erhöht ist, wobei bei einem erhöhten Risiko mehr als die Hälfte der Patienten nicht auf Clopidogrel ansprechen. Bei geringerem Risiko sind es etwa nur ein Viertel bis ein Fünftel der Patienten.

### FIGURENBESCHREIBUNG

### Figur 1

Mosaikplot für die Verteilung des Ansprechens von Patienten auf die Gabe von Clopidogrel ("Wirkung") bei INNOVANCE PFA P2Y Verschlusszeiten ("P2Y CT") ≤59 s und >59 s bei Verwendung von 3,8%igem Zitratvollblut vor der Einnahme von Clopidogrel. Die Breite bzw. Höhe der Felder zeigt den relativen Anteil der zwei Klassen an der Gesamtpopulation an.

### Figur 2

Mosaikplot für die Verteilung des Ansprechens von Patienten unter ASS-Therapie auf die Gabe von Clopidogrel ("Wirkung") bei INNOVANCE PFA P2Y Verschlusszeiten ("P2Y CT") ≤60 s und >60 s unter Verwendung von 3,2%igem Zitratvollblut vor der Einnahme von Clopidogrel. Die Breite bzw. Höhe der Felder zeigt den relativen Anteil der zwei Klassen an der Gesamtpopulation an.

### BEISPIELE

### Studiendesign

143 Patienten mit kardiovaskulären Erkrankungen, die für das operative Einsetzen eines Stents vorgesehen waren, wurde vor und 6 bis 24 Stunden nach der dazu üblichen Einnahme von 300 oder 600 mg Clopidogrel (Ladedosis) Blut entnommen.

Die Patienten hatten weder eine Krankengeschichte noch Laborergebnisse, die auf eine durch intrinsische Thrombozytendefekte, durch Defekte des VWF (VWD) oder durch die Einnahme von anderen Thromboyzytenaggregationshemmern als Acetylsalicylsäure verursachte Thrombozytendysfunktion hinwiesen. Der Großteil der Patienten nahm zum Zeitpunkt der Blutentnahmen bereits 81 oder 100 mg Acetylsalicylsäure (ASS) täglich ein.

Die Bestimmung der Thrombozytenaktivität in den Patientenproben erfolgte mit dem Platelet Function Analyzer System (PFA-100®, Siemens Healthcare Diagnostics Products GmbH, Marburg, Deutschland).

Mit Hilfe des PFA-Systems wird in Vollblutproben die primäre Hämostase unter Flussbedingungen und damit in Gegenwart von hohen Scherkräften gemessen. Als Maß für die Thrombozytenfunktion wird in diesem System die Zeit gemessen, die bis zum Verschluss einer Membranöffnung in einer speziellen Messzelle benötigt wird (Verschlusszeit). Die Verschlusszeit verhält sich umgekehrt proportional zur Thrombozytenfunktion, d.h. je länger die Verschlusszeit desto geringer die Thrombozytenaktivität.

Messzellen, die mit unterschiedlichen Membrantypen ausgestattet sind, ermöglichen die Bestimmung unterschiedlicher Funktionen der Thrombozyten. Es wurden Messzellen verwendet, die mit einer Membran ausgestattet sind, die mit Kollagen (Kol) und mit ADP beschichtet ist (Kol/ADP), oder die mit Kollagen (Kol) und mit Epinephrin (Epi) beschichtet ist (Kol/Epi), oder die mit ADP und Prostaglandin E1 beschichtet ist (INNOVANCE^{®} PFA P2Y).

Für die drei verschiedenen Messzellentypen waren vorab Grenzwerte (Cut-offs) für die Verschlusszeit (CT) (in Sekunden) bestimmt worden, die die Unterscheidung zwischen einer normalen und einer verringerten Thrombozytenfunktion erlauben. Proben mit einer verringerten Thrombozytenfunktion weisen eine velängerte Verschlusszeit auf, die oberhalb des Cut-offs liegt. Proben, die nach der Verabreichung von Clopidogrel eine Verschlusszeit oberhalb des Messzellen-spezifischen Cut-offs aufwiesen, zeigten die erwünschte Wirkung der Clopidogrel-Therapie, nämlich messbare Hemmung der Thrombozytenaktivität. Proben, die hingegen nach der Verabreichung von Clopidogrel eine Verschlusszeit unterhalb des Messzellen-spezifischen Cut-offs aufwiesen, zeigten ein Wirkungsversagen der Clopidogrel-Therapie, also eine Clopidogrel-Resistenz (oder "high on-treatment platelet reactivity").

Die Cut-offs für die Verschlusszeiten der verschiedenen Messzellentypen (in Sekunden) zur Differenzierung von normaler und verringerter Thrombozytenfunktion sind in Tab. 1 zusammengefasst.

**Tabelle 1: Cut-offs der drei PFA-Messzellentyp-spezifischen Verschlusszeiten zur Bestimmung einer verringerten Thrombozytenfunktion**

| | **Kol/EPI** | **Kol/ADP** | **INNOVANCE PFA P2Y** |
|---|---|---|---|
| **Cut-off** | 165 s | 119 s | 106 s |

Die Verschlusszeitmessungen mit INNOVANCE PFA P2Y-Messzellen wurden sowohl mit 3,2 % als auch mit 3,8 % Zitratvollblut durchgeführt, während die Kol/EPI- und Kol/ADP-Verschlusszeiten nur in 3,2 % Zitratvollblut gemessen wurden. Insgesamt wurden mit Hilfe der Verschlusszeitmessungen mit INNOVANCE PFA P2Y-Messzellen nach Einnahme von Clopidogrel bei 45,8 % (3,2 % Zitratvollblut) bzw. 37,5 % (3,8 % Zitratvollblut) der untersuchten Patienten eine Clopidogrel-Resistenz festgestellt.

Es wurde beobachtet, dass die Gruppe der Patienten, bei denen durch die Clopidogrel-Therapie keine messbare Hemmung der Thrombozytenaktivität erzielt werden konnte, die also von einer Clopidogrel-Resistenz betroffen waren, gegenüber der anderen Patientengruppe, die auf die Clopidogrel-Therapie ansprach, bereits vor der Einnahme von Clopidogrel eine signifikant erhöhte Thrombozytenfunktion (in Form kurzer Verschlusszeiten) zeigte.

Die Bestimmung einer abnormal erhöhten Thrombozytenfunktion vor der Einnahme von Clopidogrel ermöglicht demnach die Vorhersage eines erhöhten Risikos einer Clopidogrel-Resistenz.

### Ermittlung der Entscheidungsgrenze

Um eine Differenzierung von abnormal erhöhter und normaler Thrombozytenfunktion vor der Einnahme von Clopidogrel und damit die Bestimmung eines erhöhten Risikos einer Clopidogrel-Resistenz zu ermöglichen, wurden die Ergebnisse der 143 Patienten für die Berechnung einer optimalen Entscheidungsgrenze verwendet. Für jeden Messzellentyp und bei INNOVANCE PFA P2Y-Messzellen zusätzlich für jede verwendete Zitratkonzentration wurde eine eigene Entscheidungsgrenze berechnet. Als Klassifizierungsgröße für die ROC-Kurvenanalyse wurde das Ansprechen auf Clopidogrel im korrespondierenden Zitratvollblut verwendet.

Die so ermittelten optimalen Entscheidungsgrenzen sind in Tabelle 2 wiedergegeben.

**Tabelle 2: Optimale Entscheidungsgrenzen (PFA-Messzellentypspezifische Verschlusszeiten) für die Vorhersage eines erhöhten Risikos für eine Clopidogrel-Resistenz.**

| | **INNOVANCE PFA P2Y** | **INNOVANCE PFA P2Y** | **Kol/EPI** | **Kol/ADP** |
|---|---|---|---|---|
| | **(mit 3,2 % Zitratblut)** | **(mit 3,8 % Zitratblut)** | **(mit 3,2 % Zitratblut)** | **(mit 3,2 % Zitratblut)** |
| **Entscheidungsgrenze** | ≤ 60 s | ≤ 59 s | ≤ 140 s | ≤ 91 s |

### Prädikitiver Wert kurzer Verschlusszeiten

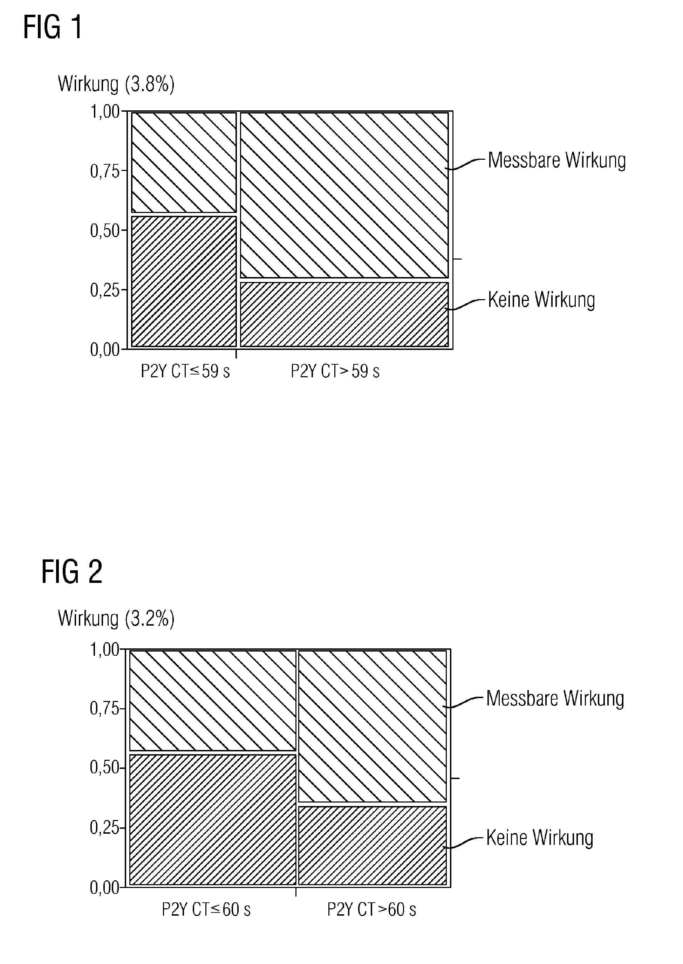

Ist die INNOVANCE PFA P2Y-Verschlusszeit eines Patienten in 3,8 % Zitratvollblut vor Einnahme von Clopidogrel kleiner als oder gleich 59 Sekunden, ist das relative Risiko, dass dieser Patient nicht auf Clopidogrel anspricht, um 98 % höher als bei Patienten mit Verschlusszeiten, die größer sind als 59 Sekunden (Relatives Risiko: 1,98; p < 0,002; Fisher's Exact Test). Die Verteilung der Ergebnisse ist in Figur 1 dargestellt.

Ist die INNOVANCE PFA P2Y-Verschlusszeit eines Patienten in 3,2 % Zitratvollblut vor Einnahme von Clopidogrel kleiner als oder gleich 60 Sekunden, ist das relative Risiko, dass dieser Patient nicht auf Clopidogrel anspricht, um 61 % höher als bei Patienten mit Verschlusszeiten, die größer sind als 60 Sekunden (Relatives Risiko: 1,61; p < 0,02; Fisher's Exact test). Die Verteilung der Ergebnisse von Patienten unter ASS-Therapie ist in Figur 2 dargestellt.

Ist die Kol/EPI-Verschlusszeit eines Patienten in 3,2 % Zitratvollblut vor Einnahme von Clopidogrel kleiner als oder gleich 140 Sekunden, ist das relative Risiko, dass dieser Patient nicht auf Clopidogrel anspricht, um 78 % höher als bei Patienten mit Verschlusszeiten, die größer sind als 140 Sekunden (Relatives Risiko: 1,78; p < 0,004; Fisher's Exact Test).

Interessanterweise ist bei einer INNOVANCE PFA P2Y-Messung nach Clopidogreleinnahme in 3,8 % Zitratvollblut das relative Risiko von Patienten nicht auf Clopidogrel anzusprechen sogar um 164 % höher, wenn die vor Einnahme von Clopidogrel in 3,2 % Zitratvollblut gemessene Kol/EPI-Verschlusszeit ≤140 Sekunden ist (Relatives Risiko: 2,64; p < 0,0001 Fisher's Exact Test).

Ist die Kol/ADP-Verschlusszeit eines Patienten unter ASS-Therapie in 3,2 % Zitratvollblut vor Einnahme von Clopidogrel kleiner als oder gleich 91 Sekunden, ist das relative Risiko, dass dieser Patient nicht auf Clopidogrel anspricht, um 47 % höher als bei Patienten mit Verschlusszeiten, die größer sind als 91 Sekunden (Relatives Risiko: 1,47; p = 0,06; Fisher's Exact Test).

## Patentansprüche

1. Verfahren zur Bestimmung des Risikos einer Clopidogrel-Resistenz eines Patienten, wobei das Verfahren folgende Schritte umfasst:
a) Messung der Thrombozytenaktivität in einer Probe des Patienten zu einem Zeitpunkt, in dem der Patient noch kein Clopidogrel eingenommen hat; und
b) Vergleich der gemessenen Thrombozytenaktivität mit einer statistisch ermittelten Entscheidungsgrenze,
wobei eine erhöhte Thrombozytenfunktion, die jenseits der Entscheidungsgrenze liegt, ein erhöhtes Risiko einer Clopidogrel-Resistenz des Patienten anzeigt.

2. Verfahren nach Anspruch 1, wobei der Patient an einer kardiovaskulären oder thrombotischen Erkrankung leidet und/oder für eine Stent-Implantation vorgesehen ist.

3. Verfahren nach einem der vorhergehenden Ansprüche, wobei die Messung der Thrombozytenaktivität in Schritt a) folgenden Schritt umfasst:
- Inkontaktbringen der Probe mit mindestens einem Thrombozytenaktivator,
bevorzugt mit mindestens einem Thrombozytenaktivator aus der Gruppe ADP, Kollagen, Epinephrin, Arachidonsäure, Ristocetin und Thrombin.

4. Verfahren nach Anspruch 3, wobei die Probe mit einer Kombination von Thrombozytenaktivatoren in Kontakt gebracht wird, bevorzugt mit Kollagen und ADP oder mit Kollagen und Epinephrin.

5. Verfahren nach einem der Ansprüche 3 und 4, wobei die Probe zusätzlich mit einem Aktivator von intrazellulären Adenylatzyklasen in Kontakt gebracht wird, bevorzugterweise aus der Gruppe Prostaglandin E1 (PGE 1), Forskolin, Prostaglandin I2, Iloprost und Cicaprost.

6. Verfahren nach einem der vorhergehenden Ansprüche, wobei die Messung der Thrombozytenaktivität in Schritt a) folgende Schritte umfasst:
- Durchleiten der Probe durch eine Kapillare und anschließend durch eine Öffnung eines Trennelements; und
- Messen der Zeit, die für die Bildung eines Thrombozytenpfropfs an der Öffnung des Trennelements bis zum Verschluss der Öffnung benötigt wird.

7. Verfahren nach Anspruch 6, wobei das Trennelement mindestens einen Thrombozytenaktivator, bevorzugt eine Kombination von Thrombozytenaktivatoren enthält.

8. Verfahren nach einem der vorhergehenden Ansprüche, wobei die Probe Vollblut oder plättchenreiches Plasma ist.

9. Verfahren nach einem der vorhergehenden Ansprüche, wobei die Messung der Thrombozytenaktivität in Schritt a) die Verwendung einer Vorrichtung umfasst, die folgende Elemente enthält:
- eine Haltekammer zum Halten der Probe;
- eine Kapillare, durch die die Probe von der Haltekammer in eine Messkammer geleitet wird;
- eine Messkammer, die durch ein Trennelement in zwei Kompartimente unterteilt ist, wobei das erste Kompartiment die Probe aus der Kapillare aufnimmt;
- ein Trennelement, das die Messkammer in zwei Kompartimente unterteilt und das eine Öffnung aufweist, durch welche die Probe aus dem ersten Kompartiment in das zweite Kompartiment fließen kann;
wobei das Trennelement mindestens einen Thrombozytenaktivator, bevorzugt eine Kombination von Thrombozytenaktivatoren enthält.
